# EUROPEAN PATENT APPLICATION

(11) **EP 4 365 582 A1**
(43) Date of publication of application: **08.05.2024**
(21) Application number: 22832939.7
(22) Date of filing: 21.06.2022
(51) Int. Cl.: G01N 27/10, C12M 1/34, G01N 15/00, G01N 27/06

(54) **FLOW CHANNEL CHIP AND SEPARATION SYSTEM**

(30) Priority: 30.06.2021 JP 2021108620
(71) Applicant: SCREEN Holdings Co., Ltd., Kyoto-shi, Kyoto 602-8585 (JP)
(72) Inventor: URAKAWA, Satoshi, Kyoto-shi, Kyoto 602-8585 (JP); SANADA, Masakazu, Kyoto-shi, Kyoto 602-8585 (JP); OSHIRO, Kyoichi, Kyoto-shi, Kyoto 606-8307 (JP)
(74) Representative: Goddar, Heinz J.
(86) International application number: PCT/JP2022/024757
(87) International publication number: WO 2023/276791

(57) **Abstract**

There is provided a flow channel chip and a separation system which enable stabilization of work for separating a specific types of dielectric particles from a suspension. The flow channel chip (10) includes: a substrate (10a) including an inlet (21) for introduction of a suspension (L1) containing a plurality of types of dielectric particles (P1, P2) including a specific type of dielectric particles (P1), and a flow channel (30) for allowing a flow of the plurality of types of dielectric particles (P1, P2) of the suspension (L1) introduced from the inlet (21); a separation electrode (50) positioned inside the flow channel (30) to cause a dielectrophoretic force for causing the specific type of dielectric particles (P1) of the plurality of types of dielectric particles (P1, P2) to move in a predetermined direction; and a sensor electrode (60) positioned inside the flow channel (30) to measure an electric resistance of the suspension (L1).

## Description

### TECHNICAL FIELD

The present disclosure relates to flow channel chips and separation systems.

### BACKGROUND ART

There is utilized dielectrophoresis as technique of controlling particles inside a micro space such as a micro fluid device. In a field of biology, cells and microorganisms are mainly subject to various research and development of property analysis, or separation and concentration. There are various ways and one examples is Patent Literature 1.

Patent Literature 1 discloses a separation device which includes a flow channel in which a sample liquid (suspension) containing circulating tumor cells (a specific type of dielectric particles) flows in a predetermined direction (fluid flow direction), a substitution part, an analyzing part, and a separation part. The separation part includes a pair of electrodes, a power supply, and a collection part. The power supply generates an alternating voltage and supplies it between the pair of electrodes. Thereby, a positive dielectrophoretic force (attractive force) acts on cancer cells and cancer cells flow along an extension direction of the pair of electrodes while being attracted to the pair of electrodes, thereby collected in the collection part.

### CITATION LIST

### PATENT LITERATURE

PATENT LITERATURE 1: JP 2017-134020 A

### SUMMARY OF INVENTION

### TECHNICAL PROBLEM

The present disclosure aims to provide a flow channel chip and a separation system which enable stabilization of work for separating a specific types of dielectric particles from a suspension.

### SOLUTION TO PROBLEM

A flow channel chip according to an aspect of the present disclosure includes a substrate, a separation electrode, and a sensor electrode. The substrate includes an inlet for introduction of a suspension containing a plurality of types of dielectric particles including a specific type of dielectric particles, and a flow channel for allowing a flow of the plurality of types of dielectric particles of the suspension introduced from the inlet. The separation electrode is positioned inside the flow channel to cause a dielectrophoretic force for causing the specific type of dielectric particles of the plurality of types of dielectric particles to move in a predetermined direction. The sensor electrode is positioned inside the flow channel to measure an electric resistance of the suspension.

A separation system according to an aspect of the present disclosure includes the aforementioned flow channel chip and a measurement device configured to measure the electric resistance of the suspension based on the output from the sensor electrode.

### ADVANTAGEOUS EFFECTS OF INVENTION

The aspects of the present disclosure enable stabilization of work for separating a specific types of dielectric particles from a suspension.

### BRIEF DESCRIPTION OF DRAWINGS

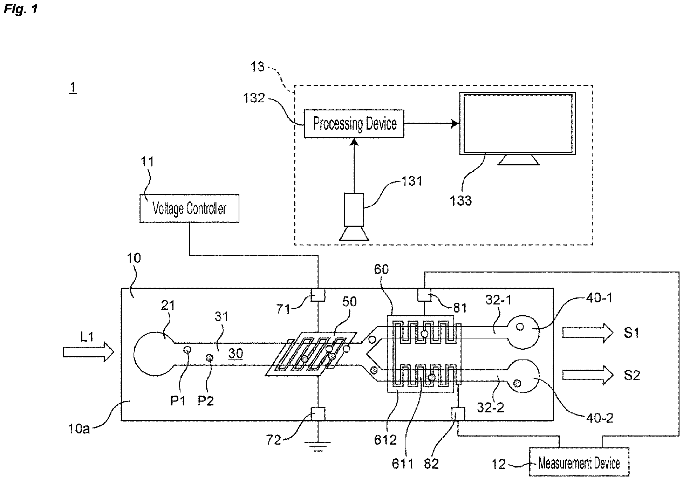

**[0009]** Fig. 1 is a block diagram of a configuration example of a separation system including a flow channel chip according to embodiment 1.
Fig. 2 is a schematic plan view of the flow channel chip of Fig. 1.
Fig. 3 is a graph indicating time variations of a resistance component and a reactance component of an impedance required for a sensor electrode.
Fig. 4 is a double logarithmic graph indicating variations of an impedance versus a frequency with regard to the different areas of the sensor electrode.
Fig. 5 is a single logarithmic graph indicating variations of a measurement efficiency versus a frequency with regard to the different areas of the sensor electrode.
Fig. 6 is a single logarithmic graph indicating a range of variations of an impedance versus a frequency with the sensor electrode having the small area.
Fig. 7 is a single logarithmic graph indicating a range of variations of an impedance versus a frequency with the sensor electrode having the large area.
Fig. 8 is a schematic plan view of a configuration example of a flow channel chip according to embodiment 2.
Fig. 9 is a schematic plan view of a configuration example 1 of a flow channel chip according to embodiment 3.
Fig. 10 is a schematic plan view of a configuration example 2 of the flow channel chip according to embodiment 3.
Fig. 11 is a schematic plan view of a configuration example 1 of a flow channel chip according to embodiment 4.
Fig. 12 is a schematic plan view of a configuration example 2 of the flow channel chip according to embodiment 4.
Fig. 13 is a schematic plan view of a configuration example 3 of the flow channel chip according to embodiment 4.

### DESCRIPTION OF EMBODIMENTS

### [1. EMBODIMENTS]

### [1.1 EMBODIMENT 1]

### [1.1.1 OUTLINE]

Fig. 1 is a block diagram of a configuration example of a separation system 1 according to embodiment 1. The separation system 1 of Fig. 1 enables separation of a specific type of dielectric particles P1 from a suspension L1. The suspension L1 contains a plurality of types of dielectric particles including the specific type of dielectric particles P1. The separation system 1 of Fig. 1 utilizes a principle of dielectrophoresis to separate the specific type of dielectric particles P1 from the plurality of types of dielectric particles contained in the suspension L1. In the present embodiment, the suspension L1 is blood. The plurality of types of dielectric particles may be cells contained in blood, for example. Examples of cells contained in blood may include tumor cells and leucocytes. In the present embodiment, for making explanation easier, the suspension L1 is supposed to contain two types of dielectric particles P1, P2. The specific type of dielectric particles P1 are tumor cells, in particular, circulating tumor cells (CTC). The dielectric particles P2 are leucocytes. The separation system 1 of Fig. 1 is used for separation of circulating tumor cells (the specific type of dielectric particles P1) from blood (the suspension L1).

The separation system 1 of Fig. 1 includes a flow channel chip 10 allowing a flow of the suspension L1. The flow channel chip 10 of Fig. 1 includes a substrate 10a, a separation electrode 50, and a sensor electrode 60. The substrate 10a includes an inlet 21 for introduction of the suspension L1 containing the plurality of types of dielectric particles P1, P2 including the specific type of dielectric particles P1, and a flow channel 30 for allowing a flow of the plurality of types of dielectric particles P1, P2 of the suspension L1 introduced from the inlet 21. The separation electrode 50 is positioned inside the flow channel 30 to cause a dielectrophoretic force for causing the specific type of dielectric particles P1 of the plurality of types of dielectric particles P1, P2 to move in a predetermined direction. The sensor electrode 60 is positioned inside the flow channel 30 to measure an electric resistance of the suspension L1.

According to the flow channel chip 10 of Fig. 1, the separation electrode 50 causes the dielectrophoretic force and the separation electrode 50 therefore can cause the specific type of dielectric particles P1 of the plurality of types of dielectric particles P1, P2 to move in the predetermined direction, thereby enabling separation of the specific type of dielectric particles P1 from the suspension L1. Intensity of the dielectrophoretic force depends on an electric field intensity produced in the suspension L1. The electric field intensity produced in the suspension L1 is determined by an impedance (a reactance and a resistance) of the separation electrode 50 and an electric resistance of the suspension L1. Influence of the electric resistance of the suspension L1 is relatively high. A change in the electric resistance of the suspension L1 causes the electric field intensity, and this results in a change in the intensity of the dielectrophoretic force. When the intensity of the dielectrophoretic force changes, separation of the specific type of dielectric particles P1 from the suspension L1 does not work in some cases. To address this situation, the flow channel chip 10 of Fig. 1 includes the sensor electrode 60 and the sensor electrode 60 enables measurement of the electric resistance of the suspension L1. Therefore, according to the flow channel chip 10, it is possible to perform work for separating the specific type of dielectric particles P1 from the suspension L1 in consideration of the electric resistance of the suspension L1. For example, it enables adjusting a voltage applied to the separation electrode 50 depending on a measured value of the electric resistance of the suspension L1 by the sensor electrode 60, or adjusting components of the suspension L1 supplied to the flow channel chip 10. When the measured value of the electric resistance of the suspension L1 by the sensor electrode 60 is an abnormal value, the work for separation can be terminated. Therefore, the flow channel chip 10 enables stabilization of work for separating the specific type of dielectric particles P1 from the suspension L1.

### [1.1.2 DETAILS]

Hereinafter, the flow channel chip 10 and the separation system 1 according to embodiment 1 will be described in detail. As shown in Fig. 1, the separation system 1 includes the flow channel chip 10, a voltage controller 11, a measurement device 12, and an analyzer 13.

### [1.1.2.1 FLOW CHANNEL CHIP]

The flow channel chip 10 forms a micro flow channel for separation of the specific type of dielectric particles P1 from the suspension L1. In the separation system 1, the suspension L1 is made to flow into the flow channel chip 10 and, inside the flow channel chip 10, the specific type of dielectric particles P1 is separated from the suspension L1. In the separation system 1, to prevent contamination, the flow channel chip 10 is constructed to be disposable.

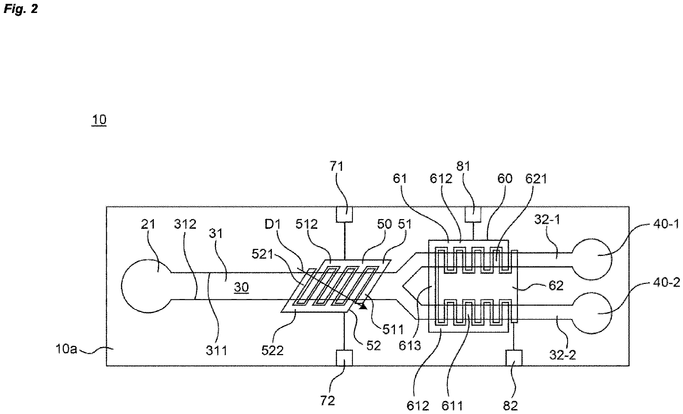

Fig. 2 is a schematic plan view of the flow channel chip 10. The flow channel chip 10 of Fig. 2 includes the substrate 10a, the separation electrode 50, the sensor electrode 60, separation electrode pads 71, 72, and sensor electrode pads 81, 82.

The substrate 10a includes the inlet 21, the flow channel 30, a plurality of collection parts 40-1, 40-2 (hereinafter, collectively designated by reference sign 40). The substrate 10a is a part defining an outer shape of the flow channel chip 10. In the present embodiment, the substrate 10a has a rectangular plate shape. The substrate 10a may be made of a glass or silicone (e.g., polydimethylsiloxane (PDMS)) substrate board or the like, for example. As one example, the substrate 10a may be constituted by a first substrate board (e.g., a glass substrate board) and a second substrate board (e.g., a PDMS substrate board) attached to each other. The first substrate board has a surface where the separation electrode 50, the sensor electrode 60, the separation electrode pads 71, 72, and the sensor electrode pads 81, 82 are formed. The second substrate board has a surface where spaces respectively corresponding to the inlet 21, the flow channel 30, and the plurality of collection parts 40-1, 40-2 are formed.

As shown in Fig. 1, the inlet 21 is a hollow part formed in the substrate 10a for allowing introduction of the suspension L1. The inlet 21 is, for example, coupled to a supplier of the suspension L1 by a tube, and is supplied with the suspension L1 via the tube.

The flow channel 30 is a hollow part formed in the substrate 10a to allow a flow of the plurality of types of dielectric particles P1, P2 of the suspension L1 introduced from the inlet 21. As shown in Fig. 2, the flow channel 30 includes a main flow channel 31 and a plurality of (two in the illustrated example) branch flow channels 32-1, 32-2 (hereinafter, collectively designated by reference sign 32).

The main flow channel 31 of Fig. 2 forms a main part of the flow channel 30. The main flow channel 31 is a part of the flow channel 30, which is used for separating the specific type of dielectric particles P1. In the present embodiment, the main flow channel 31 has a rectangular shape in its plan view. A first end (the left end in Fig. 2) in a length direction of the main flow channel 31 is connected to the inlet 21. A second end (right end in Fig. 2) of the length direction of the main flow channel 31 is connected to each branch flow channel 32.

The plurality of branch flow channels 32-1, 32-2 are parts of the flow channel 30 for transferring the dielectric particles P1, P2 with the dielectric particles P1, P2 separated from each other in the main flow channel 31 are not mixed again. The plurality of branch flow channels 32-1, 32-2 are positioned on an opposite side of the main flow channel 31 from the inlet 21. Therefore, each branch flow channel 32 is positioned downstream of the main flow channel 31 (in a right side in Fig. 2). The branch flow channel 32-1 is connected to a first end 311 in a width direction (an upward/downward direction in Fig. 2) of the main flow channel 31. As shown in Fig. 1, the branch flow channel 32-1 is part where the specific type of dielectric particles P1 separated in the main flow channel 31 flows. The branch flow channel 32-2 is connected to a second end 312 in the width direction of the main flow channel 31. As shown in Fig. 2, the branch flow channel 32-1 is part where the dielectric particles P2 which remain after separation of the specific type of dielectric particles P1 from the plurality of types of dielectric particles P1, P2 flow.

The plurality of collection parts 40 includes a first collection part 40-1 and a second collection part 40-2. The first collection part 40-1 is connected to the first end 311 in the width direction, of the main flow channel 31 via the branch flow channel 32-1. The first collection part 40-1 is for collection of the specific type of dielectric particles P1 flowing in the branch flow channel 32-1. The first collection part 40-1 may include, for example, an outlet and thereby allow supply of solution S1 containing the specific type of dielectric particles P1 from the flow channel chip 10 to an outside thereof. The second collection part 40-2 is connected to the second end 312 in the width direction W1, of the main flow channel 31 via the branch flow channel 32-2. The second collection part 40-2 is for collection of the dielectric particles P2 flowing in the branch flow channel 32-2. The second collection part 40-2 may include, for example, an outlet and thereby allow supply of solution S2 containing the dielectric particles P2 from the flow channel chip 10 to an outside.

The separation electrode 50 is positioned inside the flow channel 30 to cause the dielectrophoretic force for causing the specific type of dielectric particles P1 of the plurality of types of dielectric particles P1, P2 to move in a predetermined direction. In Fig. 2, not a whole but part of the separation electrode 50 is positioned inside the flow channel 30. In the present embodiment, the predetermined direction is a direction toward the first end 311 along the width direction the main flow channel 31. In the present embodiment, as shown in Fig. 2, the separation electrode 50 is positioned at the main flow channel 31. The separation electrode 50 is placed in or on a bottom surface of the main flow channel 31. The separation electrode 50 may be formed as a flat electrode which is relatively inexpensive, for example. As one example, the separation electrode 50 may be configured to include an electrode layer formed on the first substrate of the substrate 10a and a protective layer formed on the electrode layer. Examples of material of the electrode layer may include metal such as aluminum. The protective layer may be made of silicon oxide.

The separation electrode 50 is a comb electrode including a plurality of teeth 511, 521 arranged in the predetermined direction D1 at the bottom surface of the main flow channel 31. The predetermined direction D1 corresponds to a direction of the dielectrophoretic force made to act on the dielectric particles P1. The predetermined direction D1 is, for example, a direction (lower right direction in Fig. 2) from the first end 311 toward the second end 312 in the width direction of the flow channel 30 along a direction in which the suspension L1 flows inside the flow channel 30 (a left/right direction in Fig. 2). The plurality of teeth 511, 521 include a plurality of first teeth 511 and a plurality of second teeth 521. The first teeth 511 and the second teeth 521 are arranged alternately. The plurality of first teeth 511 extends from the first end 311 toward the second end 312 in the width direction of the main flow channel 31. The plurality of second teeth 521 extends from the second end 312 toward the first end 311 in the width direction of the main flow channel 31.

The separation electrode 50 of Fig. 2 includes a pair of patterned electrodes 51, 52. The patterned electrode 51 includes the plurality of first teeth 511 and a connector 512 positioned on a side of the first end 311 in the width direction of the main flow channel 31 to interconnect base parts of the plurality of teeth 511. The connector 512 is outside the flow channel 30. The patterned electrode 52 includes the plurality of second teeth 521 and a connector 522 positioned on a side of the second end 312 in the width direction of the main flow channel 31 to interconnect base parts of the plurality of second teeth 521. The connector 522 is outside the flow channel 30.

The sensor electrode 60 is a separate electrode from the separation electrode 50. The sensor electrode 60 is on an opposite side of the separation electrode 50 from the inlet 21 of the flow channel 30. In the present embodiment, the sensor electrode 60 is provided to span over the plurality of branch flow channels 32-1, 32-2. The sensor electrode 60 is positioned inside the flow channel 30 to measure the electric resistance of the suspension L1. In Fig. 2, not a whole but part of the sensor electrode 60 is inside the flow channel 30. Note that, in the present embodiment, the electric resistance of the suspension L1 is considered to include an impedance, or at least one of a resistance or a reactance.

The sensor electrode 60 is placed on or in the bottom surface of the branch flow channel 32. The sensor electrode 60 may be formed as a flat electrode which is relatively inexpensive, for example. As one example, the sensor electrode 60 may be configured to include an electrode layer formed on the first substrate of the substrate 10a and a protective layer formed on the electrode layer. Examples of material of the electrode layer may include metal such as aluminum. The protective layer may be made of silicon oxide.

As shown in Fig. 2, the sensor electrode 60 is a comb electrode including a plurality of teeth 611, 621 arranged along the length direction of the flow channel 30 at the bottom surface of the flow channel 30. In the present embodiment, the plurality of teeth 611, 621 are arranged at each branch flow channel 32 of the flow channel 30 along a length direction of each branch flow channel 32. The plurality of teeth 611, 621 include a plurality of first teeth 611 and a plurality of second teeth 621. The first teeth 611 and the second teeth 621 are arranged alternately. In the first branch flow channel 32-1, the plurality of first teeth 611 extend from one side further from the second branch flow channel 32-2, of the first branch flow channel 32-1 toward another side closer to the second branch flow channel 32-2, of the first branch flow channel 32-1. The plurality of second teeth 621 extend from one side closer to the second branch flow channel 32-2, of the first branch flow channel 32-1 toward another side further from the second branch flow channel 32-2, of the first branch flow channel 32-1. In the second branch flow channel 32-2, the plurality of first teeth 611 extends from one side further from the first branch flow channel 32-1, of the second branch flow channel 32-2 toward another side closer to the first branch flow channel 32-1, of the second branch flow channel 32-2. The plurality of second teeth 621 extend from one side closer to the first branch flow channel 32-1, of the second branch flow channel 32-2 toward another side further from the first branch flow channel 32-1, of the second branch flow channel 32-2.

The sensor electrode 60 of Fig. 2 includes a pair of patterned electrodes 61, 62. The patterned electrode 61 includes the plurality of first teeth 611 positioned at the respective first and second branch flow channels 32-1, 32-2, a connector 612 interconnecting base parts of the plurality of first teeth 611 positioned at the first branch flow channel 32-1, a connector 612 interconnecting base parts of the plurality of first teeth 611 positioned at the second branch flow channel 32-2, and a coupler coupling the connectors 612 one other. Each connector 612 is outside the flow channel 30. The second patterned electrode 62 includes the plurality of second teeth 621 positioned at the respective first and second branch flow channels 32-1, 32-2, and a connector 622 between the first and second branch flow channels 32-1, 32-2 to interconnect base parts of the plurality of second teeth 621.

Measuring the electric resistance of the suspension L1 using the sensor electrode 60 includes measuring a current flowing through the suspension L1 between the first and second patterned electrodes 61, 62 of the sensor electrode 60 with a measurement voltage being applied between the first and second patterned electrodes 61, 62 of the sensor electrode 60. The measurement voltage is, for example, an alternating voltage with a predetermined frequency. This enables measurement of an impedance between the first and second patterned electrodes 61, 62 of the sensor electrode 60. The impedance between the first and second patterned electrodes 61, 62 of the sensor electrode 60 includes a reactance component (imaginary part) and a resistance component (real part). Mainly, the reactance component is determined by the sensor electrode 60, and the resistance component is determined by the electric resistance of the suspension L1. In detail, the resistance component includes an electric resistance of the sensor electrode 60. Therefore, it is possible to calculate the electric resistance of the suspension L1 from the impedance between the first and second patterned electrodes 61, 62 of the sensor electrode 60 relative to the measurement voltage. Note that, the predetermined frequency of the measurement voltage may be a specific frequency or include frequencies within a certain range. When the predetermined frequency of the measurement voltage includes frequencies within a certain range, measurement using a saw wave, a triangle wave, or the like may be conducted.

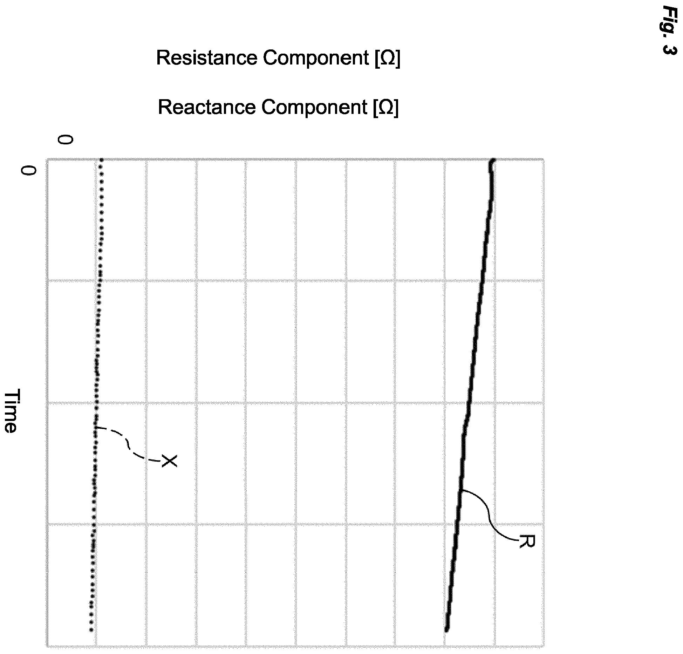

Fig. 3 is a graph indicating time variations of the resistance component and the reactance component of the impedance determined by the sensor electrode 60 (the impedance between the first and second patterned electrodes 61, 62 of the sensor electrode 60). Fig. 3 shows a result of measurement of the impedance for a predetermined period of time. The predetermined period of time is, for example, 2 hours. In Fig. 3, X indicates the reactance component and R indicates the resistance component. As apparent from Fig. 3, there is no substantial change in the reactance component X but the resistance component R changes with time. Especially, in Fig. 3, the resistance component R decreases with time.

The separation electrode pads 71, 72 are used to apply the predetermined voltage across the separation electrode 50. The separation electrode pads 71, 72 are connected to the pair of patterned electrodes 51, 52 of the separation electrode 50, respectively in order to enable application of the predetermined voltage between the pair of patterned electrodes 51, 52 of the separation electrode 50. The separation electrode pads 71, 72 are connected to the pair of patterned electrodes 51, 52 of the separation electrode 50, respectively via patterned wiring, for example. The separation electrode pads 71, 72 are placed on an outer surface of the substrate 10a. Controlling a voltage between the separation electrode pads 71, 72 enables controlling a voltage across the separation electrode 50.

The sensor electrode pads 81, 82 are provided separately from the separation electrode pads 71, 72. The sensor electrode pads 81, 82 are used to apply the measurement voltage across the sensor electrode 60. The sensor electrode pads 81, 82 are connected to the first and second patterned electrodes 61, 62 of the sensor electrode 60, respectively in order to enable application of the measurement voltage between the first and second patterned electrodes 61, 62 of the sensor electrode 60. The sensor electrode pads 81, 82 are connected to the pair of patterned electrodes 61, 62 of the sensor electrode 60, respectively via patterned wiring. The sensor electrode pads 81, 82 are placed on an outer surface of the substrate 10a. Controlling a voltage between the sensor electrode pads 81, 82 enables controlling a voltage across the sensor electrode 60.

As shown in Fig. 1 and Fig. 2, the flow channel chip 10 includes the sensor electrode 60 for measurement of the electric resistance of the suspension L1. In the flow channel chip 10, the separation electrode 50 and the sensor electrode 60 are separate electrodes. Therefore, it is possible to design the separation electrode 50 and the sensor electrode 60 in accordance with their respective conditions suitable for their respective purposes. For example, when the separation electrode 50 is used as the sensor electrode 60, the structure is limited by the condition required for the separation electrode 50 to make the dielectrophoretic force act on the specific type of dielectric particles P1. However, in the present embodiment, since the sensor electrode 60 is a separate electrode from the separation electrode 50, the structure of the sensor electrode 60 is not limited by the conditions required for the separation electrode 50. Accordingly, there is no need for the sensor electrode 60 to depend on the structure of the separation electrode 50 and thus a detection sensitivity of the sensor electrode 60 can be adjusted by a high freedom of design. As a result, it is possible to improve stabilization of work for separating the specific types of dielectric particles P1.

Hereinafter, the structure of the sensor electrode 60 will be described further. In the present embodiment, an area of part positioned inside the flow channel 30, of the sensor electrode 60 is larger than an area of part positioned inside the flow channel 30, of the separation electrode 50. In the case according to the present embodiment, the area of part positioned inside the flow channel 30, of the sensor electrode 60 is a sum of areas of parts positioned inside the flow channel 30, of the plurality of teeth 611, 621 of the sensor electrode 60. The area of part positioned inside the flow channel 30, of the sensor electrode 60 may be variable by dimensions (mainly, widths) of the teeth 611, 621, intervals of the teeth 611, 621, and, the numbers of teeth 611, 621. To adjust the area of part positioned inside the flow channel 30, of the sensor electrode 60, it is preferable to vary the numbers of teeth 611, 621. In the case according to the present embodiment, the area of part positioned inside the flow channel 30, of the separation electrode 50 is a sum of areas of parts positioned inside the flow channel 30, of the plurality of teeth 511, 521 of the separation electrode 50. The area of part positioned inside the flow channel 30, of the separation electrode 50 may be variable by dimensions (mainly, widths) of the teeth 511, 521, intervals of the teeth 511, 521, and, the numbers of teeth 511, 521. To adjust the area of part positioned inside the flow channel 30, of the separation electrode 50, it is preferable to vary the numbers of teeth 511, 521.

In the separation electrode 50, to reduce burden on the dielectric particles P1, it is preferable to shorten contact time between the dielectric particles P1 and the separation electrode 50. Therefore, the area of part positioned inside the flow channel 30, of the separation electrode 50 tends to be set to be smaller. In contrast, in some cases, it is preferable to increase the area of part positioned inside the flow channel 30, of the sensor electrode 60.

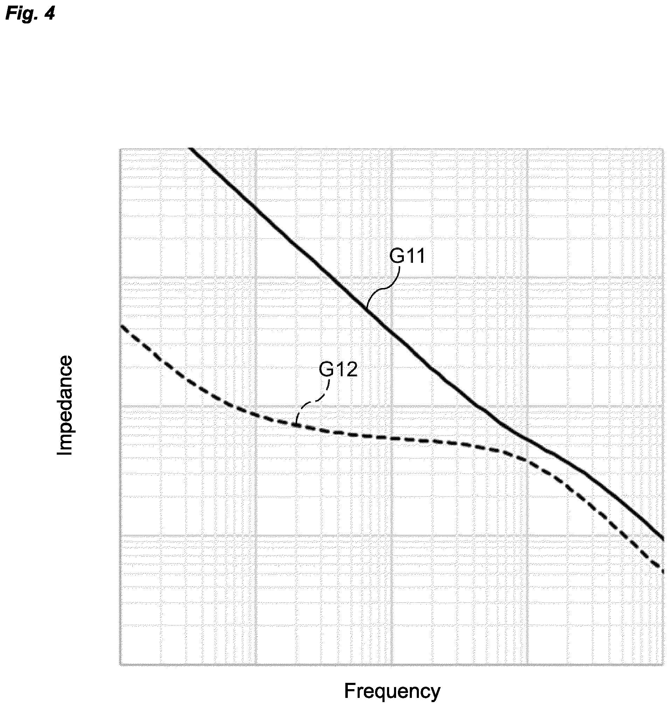

Fig. 4 is a double logarithmic graph indicating variations of an impedance versus a frequency with regard to the different areas of the sensor electrode 60. In Fig. 4, G11 corresponds to a case where the area of the sensor electrode is small, and G12 corresponds to a case where the area of the sensor electrode is large. G12 is larger in the area of part positioned inside the flow channel 30, of the sense electrode 60 than G11. As understood from Fig. 4, a difference in the area of the sensor electrode 60 causes a difference in a variation of the measured value of the impedance versus the frequency of the measurement voltage.

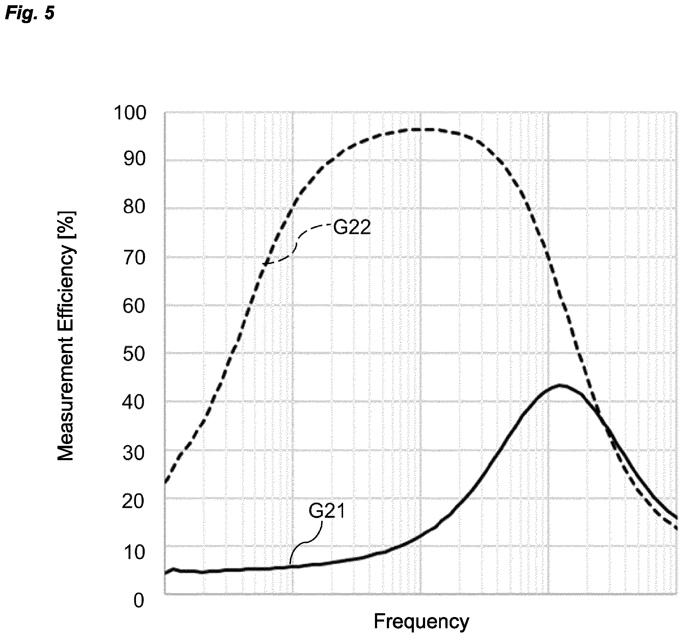

Fig. 5 is a single logarithmic graph indicating variations of a measurement efficiency versus a frequency with regard to the different areas of the sensor electrode 60. The measurement efficiency is a percentage of the electric resistance of the suspension L1 to the measured value of the impedance. In Fig. 5, G21 corresponds to a case where the area of the sensor electrode is small, and G22 corresponds to a case where the area of the sensor electrode is large. As shown in Fig. 5, the maximum value of the measurement efficiency of G21 is about 40% and in contrast the maximum value of the measurement efficiency of G22 is more than 90%. The high measurement efficiency means that effects of the impedance of the sensor electrode 60 on the measured value of the impedance is low. This enables decreasing the effect caused by a variation of the performance of the sensor electrode 60, and decreasing a variation of the performance of the flow channel chip 10. Thus, it is preferable that the measurement efficiency is higher.

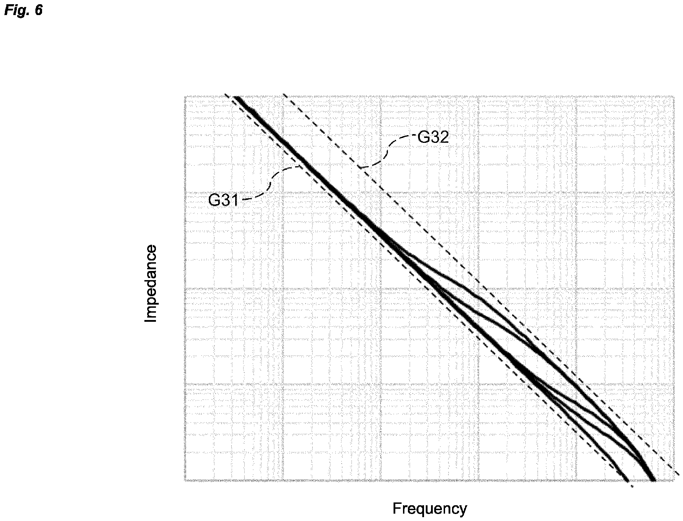

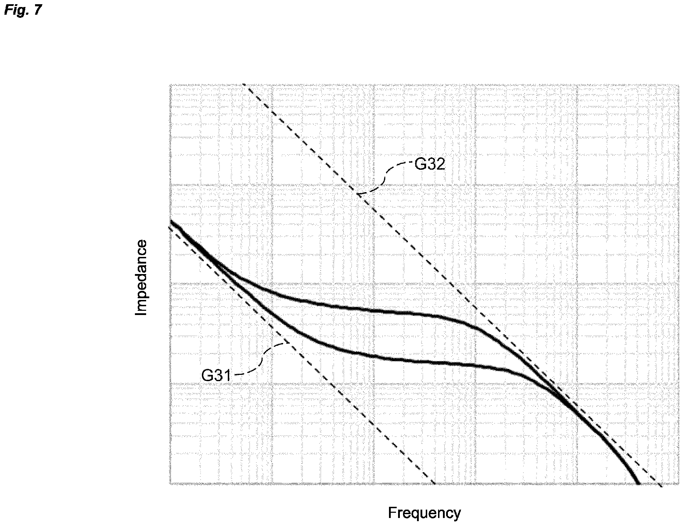

In some cases, the electric resistance of the suspension L1 may vary with time. An extent that change in the electric resistance of the suspension L1 is allowed is determined by the impedance (mainly the reactance) of the sensor electrode 60. Fig. 6 is a single logarithmic graph indicating a range of variations of an impedance versus a frequency with the sensor electrode 60 having the small area. Fig. 7 is a single logarithmic graph indicating a range of variations of an impedance versus a frequency with the sensor electrode 60 having the large area. Change in the electric resistance of the suspension L1 causes change in the measured value of the impedance versus the frequency. This is reflected on upward and downward movement of the graph in Fig. 6 and Fig. 7. An extent that the graph can vary is determined by the impedance of the sensor electrode 60. In Fig. 6 and Fig. 7, regions between dotted lines G31, G32 indicate a range in which the graph of the measured value of the impedance versus the frequency is variable, and the dotted lines G31, G32 are determined by the impedance of the sensor electrode 60. The dotted lines G31, G32 are not affected by change in the electric resistance of the suspension L1. With an increase in a distance between the dotted lines G31, G32, and an increase in a variable range of the measured value of the impedance in a frequency direction, the measurement efficiency of the electric resistance of the suspension L1 improves, and a frequency range available for measurement becomes wider. As apparent from Fig. 6 and Fig. 7, when the area of the sensor electrode 60 is large, the frequency range available for measurement is wide, and it may be possible to read not less than one digit change in the electric resistance of the suspension L1 at a certain frequency. In contrast, when the area of the sensor electrode 60 is small, the frequency range available for measurement is narrow, and the performance of measurement by the sensor electrode 60 is poor relative to the case where the area of the sensor electrode 60 is large.

Accordingly, it is preferable that the area of the part positioned inside the flow channel 30, of the sensor electrode 60 is larger. Therefore, in the present embodiment, the area of part positioned inside the flow channel 30, of the sensor electrode 60 is set to be larger than the area of part positioned inside the flow channel 30, of the separation electrode 50. This enables reduction of burden on the dielectric particles P1 applied by the separation electrode 50 and enables improvement of the performance of measurement by the sensor electrode 60.

As described above, it is preferable that the measurement efficiency (the percentage of the electric resistance of the suspension L1 relative to the measured value of the impedance) is higher. Thus, the reactance of the sensor electrode 60 is set to give the measurement efficiency which is equal to or larger than a prescribed value. In the present embodiment, the reactance of the sensor electrode 60 is smaller than a lower limit of a measurable range of the electric resistance of the suspension L1. For example, the reactance of the sensor electrode 60 may be equal to or smaller than one-fifth of the lower limit of the measurable range of the electric resistance of the suspension L1. The reactance of the sensor electrode 60 is a value involving the frequency of the measurement voltage. The measurable range of the electric resistance of the suspension L1 is appropriately set based on properties of the suspension L1 supplied to the flow channel chip 10. For example, when the measurable range of the electric resistance of the suspension L1 is a range of about 700Ω to 1000Ω, the reactance of the sensor electrode 60 is set to about 100Ω.

### [1.1.2.2 VOLTAGE CONTROLLER]

The voltage controller 11 of Fig. 1 is configured to control a voltage applied to the flow channel chip 10 in order to produce the dielectrophoretic force. In the present embodiment, the voltage controller 11 controls the voltage across the separation electrode 50 to separate the specific type of dielectric particles P1 from the plurality of types of dielectric particles P1, P2 contained in the suspension L1. The voltage controller 11 includes, for example, a function generator. The voltage controller 11 of Fig. 1 is connected to the separation electrode 50 via the separation electrode pads 71, 72. The voltage controller 11 controls the voltage across the separation electrode 50 for causing the dielectrophoretic force causing the specific type of dielectric particles P1 of the plurality of types of dielectric particles P1, P2 to move in the predetermined direction. In the present embodiment, the voltage controller 11 is configured to apply the predetermined voltage across the separation electrode 50 via the separation electrode pads 71, 72. The predetermined voltage is, for example, an alternating voltage. A frequency of the predetermined voltage is set to allow the separation electrode 50 to produce the positive dielectrophoretic force acting on the specific type of dielectric particles P1. In this case, the frequency is set so that no dielectrophoretic force acts on dielectric particles P2 other than the specific type of dielectric particles P1 of the plurality of types of dielectric particles P1, P2 or the dielectrophoretic force is relatively small even when the positive or negative dielectrophoretic force (attractive force or repulsive force) acts thereon. The frequency of the predetermined voltage is, for example, 600 kHz.

### [1.1.2.3 MEASUREMENT DEVICE]

The measurement device 12 of Fig. 1 is configured to measure the electric resistance of the suspension L1 based on output from the sensor electrode 60. In the present embodiment, the measurement device 12 is configured to control the voltage across the sensor electrode 60 to measure the electric resistance of the suspension L1. The measurement device 12 includes, for example, a combination of a signal generator such as a function generator and a measuring instrument such as an oscilloscope, or, a source/measure unit. The measurement device 12 of Fig. 1 is connected to the sensor electrode 60 via the sensor electrode pads 81, 82. The measurement device 12 measures a current flowing between the first and second patterned electrodes 61, 62 of the sensor electrode 60 as the output from the sensor electrode 60 while applying the measurement voltage between the first and second patterned electrodes 61, 62 of the sensor electrode 60 via the sensor electrode pads 81, 82. From the measurement voltage and the output from the sensor electrode 60, the impedance between the first and second patterned electrodes 61, 62 of the sensor electrode 60 is determined. The impedance between the first and second patterned electrodes 61, 62 of the sensor electrode 60 includes the reactance component determined by the sensor electrode 60 and the resistance component determined by the electric resistance of the suspension L1. Therefore, the measurement device 12 can determine the electric resistance of the suspension L1 from the impedance between the first and second patterned electrodes 61, 62 of the sensor electrode 60 versus the measurement voltage.

### [1.1.2.4 ANALYZER]

The analyzer 13 of Fig. 1 is configured to perform analysis regarding separation of the specific type of dielectric particles P1 from the plurality of types of dielectric particles P1, P2 contained in the suspension L1. The analyzer 13 of Fig. 1 includes an imaging device 131, a processing device 132, and a display device 133.

The imaging device 131 is configured to take an image of target part in the flow channel chip 10. The target part is, for example, the main flow channel 31. From an image of the main flow channel 31, it is possible to obtain tracks of movement of the dielectric particles P1, P2 passing through the flow channel 30. The imaging device 131 includes, for example, a camera including an imaging device such as a CCD image sensor or a CMOS image sensor, and an optical microscope module. The optical microscope module may be a phase contrast microscope, or a reflected light microscope. Further, the optical microscope module may be configured to be switchable between a phase contrast microscope and a reflected light microscope by replacing lenses, for example. Note that, to conduct fluorescence microscopy, fluorescence filters may be used appropriately. Operations of the imaging device 131 may be controlled by the processing device 132.

The display device 133 is configured to display information from the processing device 132. The display device 133 is, for example, a liquid crystal display or an organic EL display.

The processing device 132 is configured to control operations of the analyzer 13. The processing device 132 may be realized by, for example, a computer system including one or more processors (microprocessors) and one or more memories. The one or more processors performs one or more programs (stored in the one or more memories) to realize predetermined functionality. In one example, the processing device 132 performs image analysis on an image taken by the imaging device 131 to determine tracks of movement of the dielectric particles P1, P2 passing through the main flow channel 31. The processing device 132 may display the image taken by the imaging device 131 or the tracks of movement determined by the image analysis, by the display device 133.

### [1.1.3 ADVANTAGEOUS EFFECTS]

The aforementioned flow channel chip 10 includes: the substrate 10a including the inlet 21 for introduction of the suspension L1 containing the plurality of types of dielectric particles P1, P2 including the specific type of dielectric particles P1, and the flow channel 30 for allowing a flow of the plurality of types of dielectric particles P1, P2 of the suspension L1 introduced from the inlet 21; the separation electrode 50 positioned inside the flow channel 30 to cause a dieletrophoretic force for causing the specific type of dielectric particles P1 of the plurality of types of dielectric particles P1, P2 to move in the predetermined direction; and the sensor electrode 60 positioned inside the flow channel 30 to measure the electric resistance of the suspension L1. This configuration enable stabilization of work for separating the specific types of dielectric particles P1 from the suspension L1.

Further, in the flow channel chip 10, the separation electrode 50 and the sensor electrode 60 are separate electrodes. This configuration enables designing the separation electrode 50 and the sensor electrode 60 in accordance with their respective conditions suitable for their respective purposes and thus enables improvement of stabilization of work for separating the specific types of dielectric particles P1.

Further, in the flow channel chip 10, the area of part positioned inside the flow channel 30, of the sensor electrode 60 is larger than the area of part positioned inside the flow channel 30, of the separation electrode 50. This configuration enables decreasing a variation of a performance in the flow channel chip 10 and additionally enables improvement of a performance of measurement using the sensor electrode 60.

Further, in the flow channel chip 10, the reactance of the sensor electrode 60 is smaller than the lower limit of the measurable range of the electric resistance of the suspension (L1). This configuration enables decreasing a variation of a performance in the flow channel chip 10.

Further, in the flow channel chip 10, the reactance of the sensor electrode 60 is equal to or smaller than one-fifth of the lower limit of the measurable range of the electric resistance of the suspension L1. This configuration enables decreasing a variation of a performance in the flow channel chip 10 and additionally enables improvement of a performance of measurement using the sensor electrode 60.

Further, in the flow channel chip 10, the sensor electrode 60 is a comb electrode including a plurality of teeth 611, 621 arranged along the length direction of the flow channel 30 in or on the bottom surface of the flow channel 30. This configuration enables stabilization of work for separating the specific types of dielectric particles P1 from the suspension L1.

Further, in the flow channel chip 10, the sensor electrode 60 is positioned in a place on the opposite side of the separation electrode 50 from the inlet 21 in the flow channel 30. This configuration enables stabilization of work for separating the specific types of dielectric particles P1 from the suspension L1.

Further, in the flow channel chip 10, the flow channel 30 includes the plurality of branch flow channels 32 connected to the plurality of collection parts 40. The sensor electrode 60 is provided to span over the plurality of branch flow channels 32. This configuration enables measurement of the electric resistance of the entire suspensions L1 passing through the flow channel 30. For example, when a distribution of the electric resistance of the suspension L1 occurs in the width direction of the flow channel 30, the suspensions L1 flowing into the plurality of collection parts 40 may have different electric resistances. The above configuration measures the electric resistance of the entire suspensions L1 passing through the flow channel 30. This enables reduction of an effect due to such a distribution of the electric resistance of the suspension L1 and thus enable more accurate measurement of the electric resistance of the suspension L1. Further, it is possible to perform appropriate feedback to external devices such as the voltage controller 11, the measurement device 12, and the analyzer 13.

Further, in the separation system 1, the flow channel chip 10 is disposable. This configuration enables prevention of contamination and additionally improvement of usability of the separation system 1.

### [1.2 EMBODIMENT 2]

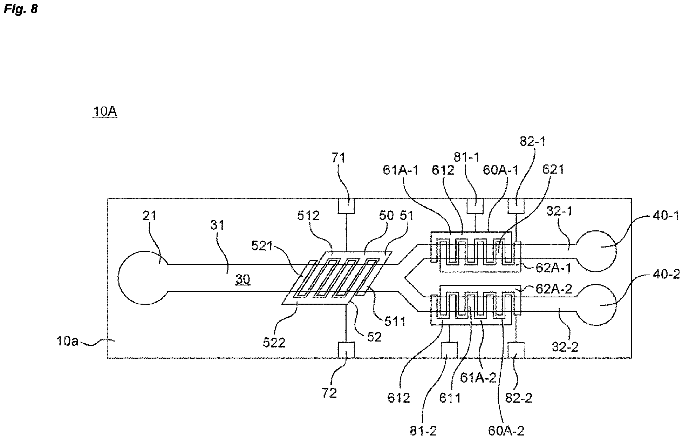

Fig. 8 is a schematic plan view of a configuration example of a flow channel chip according to embodiment 2. Hereinafter, the flow channel chip of Fi. 8 is designated by reference sign 10A. The flow channel chip 10A of Fig. 8 includes a plurality of sensor electrodes 60A-1, 60A-2 (hereinafter, collectively designated by reference sign 60A). The plurality of sensor electrodes 60A-1, 60A-2 are provided to the plurality of branch flow channels 32-1, 32-2, respectively.

As shown in Fig. 8, the sensor electrode 60A-1 is a comb electrode including the plurality of teeth 611, 621 arranged along the length direction of the flow channel 30 at the bottom surface of the flow channel 30. In the present embodiment, the plurality of teeth 611, 621 of the sensor electrode 60A-1 are arranged at the first branch flow channel 32-1 of the flow channel 30 along a length direction thereof. The sensor electrode 60A-1 includes a first patterned electrode 61A-1 and a second patterned electrode 62A-1. The first patterned electrode 61A-1 includes the plurality of first teeth 611 positioned at the first branch flow channel 32-1 and the connector 612 positioned outside the first branch flow channel 32-1 to interconnect base parts of the plurality of first teeth 611. The second patterned electrode 62A-1 includes the plurality of second teeth 621 positioned at the first branch flow channel 32-1 and the connector 622 positioned outside the first branch flow channel 32-1 to interconnect base parts of the plurality of second teeth 621.

As shown in Fig. 8, the sensor electrode 60A-2 is a comb electrode including the plurality of teeth 611, 621 arranged along the length direction of the flow channel 30 at the bottom surface of the flow channel 30. In the present embodiment, the plurality of teeth 611, 621 of the sensor electrode 60A-2 are arranged at the second branch flow channel 32-2 of the flow channel 30 along a length direction thereof. The sensor electrode 60A-2 includes a first patterned electrode 61A-2 and a second patterned electrode 62A-2. The first patterned electrode 61A-2 includes the plurality of first teeth 611 positioned at the second branch flow channel 32-2 and the connector 612 positioned outside the second branch flow channel 32-2 to interconnect base parts of the plurality of first teeth 611. The second patterned electrode 62A-2 includes the plurality of second teeth 621 positioned at the second branch flow channel 32-2 and the connector 622 positioned outside the second branch flow channel 32-2 to interconnect base parts of the plurality of second teeth 621.

The flow channel chip 10A of Fig. 8 includes the sensor electrode pads 81-1, 82-1 connected to the sensor electrode 60A-1 and the sensor electrode pads 81-2, 82-2 connected the sensor electrode 60A-2. The plurality of sensor electrodes 60A can be applied with voltage individually.

As described above, in the flow channel chip 10A, the flow channel 30 includes the plurality of branch flow channels 32 connected to the plurality of collection parts 40. The sensor electrode 60A is provided to each of the plurality of branch flow channels 32. This configuration enables measuring the electric resistances of the suspensions L1 respectively passing through the plurality of branch flow channels 32. For example, when a distribution of the electric resistance of the suspension L1 occurs in the width direction of the flow channel 30, in some cases the suspensions L1 flowing into the plurality of collection parts 40 may have different electric resistances. The aforementioned configuration measures a resistance of the suspension L1 passing through each branch flow channel 32. It is possible to reduce an effect due to such a distribution of the electric resistance of the suspension L1 and thus it is possible to conduct more accurate measurement of the electric resistance of the suspension L1. Further, it is possible to perform appropriate feedback to external devices such as the voltage controller 11, the measurement device 12, and the analyzer 13. Since the flow channel chip 10A includes the plurality of sensor electrodes 60A, it is possible to utilize outputs from the plurality of sensor electrodes 60A. For example, it is possible to determine the electric resistance of the suspension L1 by use of an average of the outputs from the plurality of sensor electrodes 60A. In this case, it is possible to reduce an effect due to a variation of the sensor electrode 60A. Further, even when one or some of the plurality of sensor electrodes 60A is broken, the electric resistance of the suspension L1 can be still determined.

### [1.3 EMBODIMENT 3]

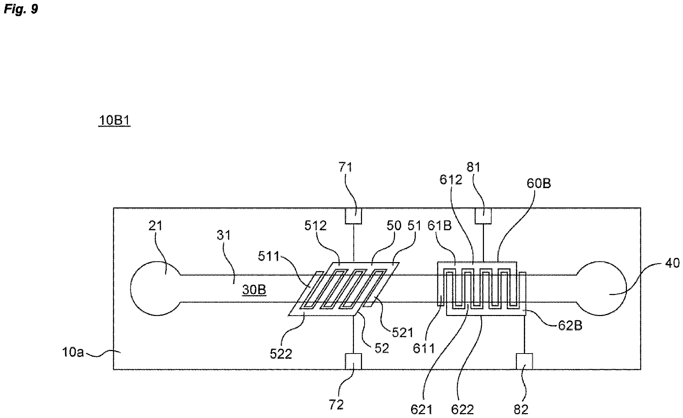

Fig. 9 is a schematic plan view of a configuration example 1 of a flow channel chip according to embodiment 3. Hereinafter, the flow channel chip of Fig. 9 is denoted by reference sign 10B1. The flow channel chip 10B1 of Fig. 9 includes a flow channel 30B different from the flow channel 30 of the flow channel chip 10 of embodiment 1. The flow channel 30B of Fig. 9 does not include the branch flow channel 32 as described in embodiment 1, but is constituted by the main flow channel 31. The first end (the left end in Fig. 9) in the length direction (the left/right direction in Fig. 9) of the main flow channel 31 is connected to the inlet 21. The second end (the right end in Fig. 9) in the length direction of the main flow channel 31 is connected to the collection part 40.

The flow channel chip 10B1 of Fig. 9 includes a sensor electrode 60B different from the sensor electrode 60 of the flow channel chip 10 of embodiment 1. The sensor electrode 60B of Fig. 9 is positioned in a place on the opposite side of the separation electrode 50 from the inlet 21. In more detail, the sensor electrode 60B is positioned in a place between the separation electrode 50 and the collection part 40 in the main flow channel 31. As shown in Fig. 9, the sensor electrode 60B is a comb electrode including the plurality of teeth 611, 621 arranged along the length direction of the main flow channel 31 at a bottom surface of the main flow channel 31. The sensor electrode 60B includes a first patterned electrode 61B and a second patterned electrode 62B. The first patterned electrode 61B includes the plurality of first teeth 611 positioned at the main flow channel 31 and the connector 612 positioned outside the main flow channel 31 to interconnect base parts of the plurality of first teeth 611. The second patterned electrode 62B includes the plurality of second teeth 621 positioned at the main flow channel 31 and the connector 622 positioned outside the main flow channel 31 to interconnect base parts of the plurality of second teeth 621.

In the flow channel chip 10B1 of Fig. 9, the sensor electrode pads 81, 82 are connected to the first patterned electrode 61B and the second patterned electrode 62B, respectively.

Note that, in the flow channel chip 10B1, the separation electrode 50 is configured to capture the specific type of dielectric particles P1. Accordingly, the separation electrode 50 is positioned inside to cause the dielectrophoretic force causing the specific type of dielectric particles P1 of the plurality of types of dielectric particles P1, P2 to move in the predetermined direction, and the predetermined direction may be a direction approaching the separation electrode 50.

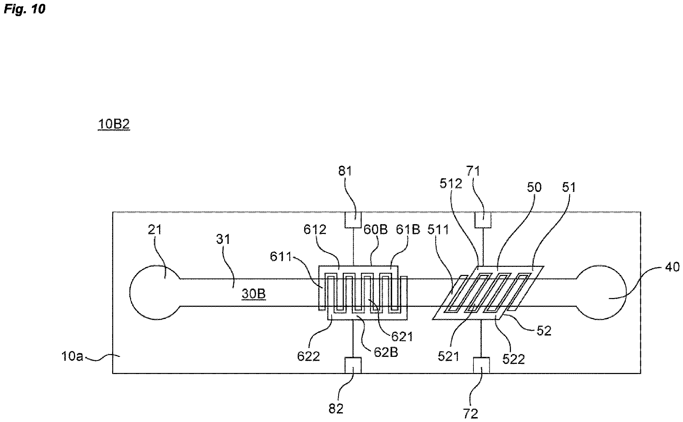

Fig. 10 is a schematic plan view of a configuration example 2 of a flow channel chip according to embodiment 3. Hereinafter, the flow channel chip of Fig. 10 is denoted by reference sign 10B2. The flow channel chip 10B2 of Fig.10 is different from the flow channel chip 10B1 of Fig. 9 in a positional relationship between the separation electrode 50 and the sensor electrode 60B. The sensor electrode 60B of Fig. 10 is positioned in not a place on the opposite side of the separation electrode 50 from the inlet 21 in the flow channel 30B but a place between the inlet 21 and the separation electrode 50 in the flow channel 30B.

In the flow channel chip according to embodiment 3, the sensor electrode 60B may be positioned in both a place between the inlet 21 and the separation electrode 50 in the flow channel 30B and a place on the opposite side of the separation electrode 50 from the inlet 21. When the plurality of sensor electrodes 60B are provided as described above, it is possible to determine the electric resistance of the suspension L1 by use of an average of the outputs from the plurality of sensor electrodes 60B, for example. This enables decreasing an effect due to a variation of the sensor electrode 60B. Further, even when one or some of the plurality of sensor electrodes 60B is broken, for example, it is possible to determine the electric resistance of the suspension L1.

As described above, the sensor electrode 60B may be positioned in at least one of a place between the inlet 21 and the separation electrode 50 or a place on an opposite side of the separation electrode 50 from the inlet 21. This configuration enables stabilization of work for separating the specific types of dielectric particles P1 from the suspension L1. This can similarly apply to embodiment 1 described above and embodiment 4 described below.

### [1.4 EMBODIMENT 4]

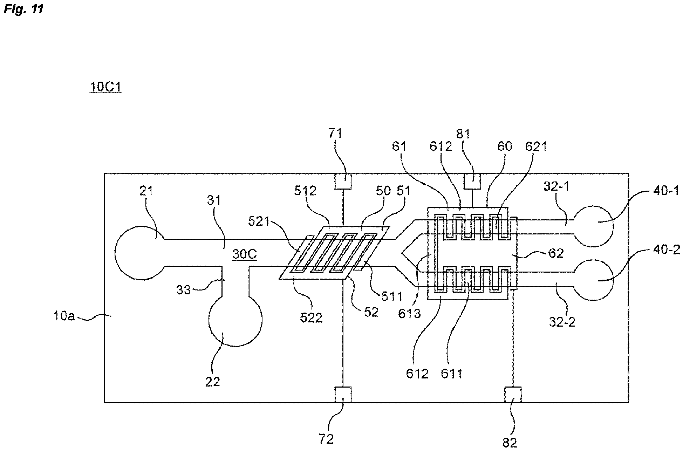

Fig. 11 is a schematic plan view of a configuration example 1 of a flow channel chip according to embodiment 4. Hereinafter, the flow channel chip of Fig. 11 is denoted by reference sign 10C1. The flow channel chip 10C1 of Fig. 11 includes a flow channel 30C different from the flow channel 30 of the flow channel chip 10 of embodiment 1. The flow channel 30C of Fig. 11 includes a connection channel 33 interconnecting the main flow channel 31 and an inlet 22. The inlet 22 is formed in the substrate 10a. The inlet 22 is part of the substrate 10a and allows introduction of a replacement solution. The replacement solution is used for removing one or more unnecessary components from the suspension L1 and carrying the plurality of types of dielectric particles P1, P2 of the suspension L1, for example. The unnecessary components are components unnecessary in separation of the specific type of dielectric particles P1 from the suspension L1. Examples of the unnecessary components include small cells P3 such as erythrocytes contained in blood, and, solutions. The replacement solution may be selected to be capable of carrying the plurality of types of dielectric particles P1, P2 but not to inhibit the dielectrophoretic force from acting on the plurality of types of dielectric particles P1, P2. Especially, to facilitate separation of the specific type of dielectric particles P1, a liquid with conductivity lower than the solution of the suspension L1 may be used as the replacement solution. Note that, the substrate 10a may be provided with an outlet for discharging the unnecessary components of the suspension L1 replaced by the replacement solution.

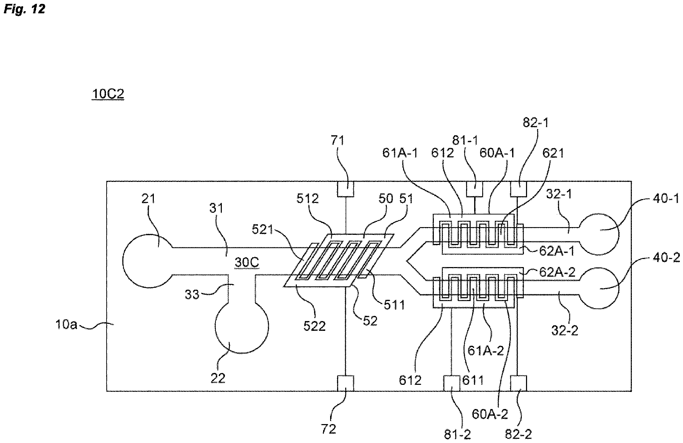

Fig. 12 is a schematic plan view of a configuration example 2 of a flow channel chip according to embodiment 4. Hereinafter, the flow channel chip of Fig. 12 is denoted by reference sign 10C2. The flow channel chip 10C2 of Fig. 12 includes a flow channel 30C different from the flow channel 30 of the flow channel chip 10A of embodiment 2. The flow channel 30C of Fig. 12 includes the connection channel 33 interconnecting the main flow channel 31 and the inlet 22.

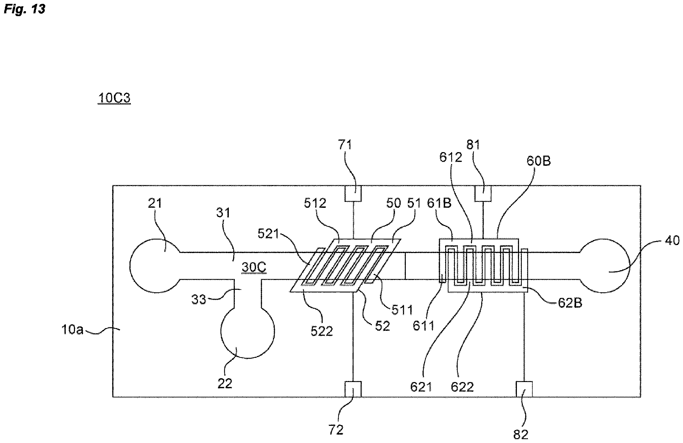

Fig. 13 is a schematic plan view of a configuration example 3 of a flow channel chip according to embodiment 4. Hereinafter, the flow channel chip of Fig. 13 is denoted by reference sign 10C3. The flow channel chip 10C3 of Fig. 13 includes a flow channel 30C different from the flow channel 30B of the flow channel chip 10B1 of embodiment 3. The flow channel 30C of Fig. 13 includes the connection channel 33 interconnecting the main flow channel 31 and the inlet 22.

As described above, in embodiment 4, the flow channel 30C may include the connection channel 33 connected to the inlet 22 for introduction of the replacement solution. This enables removal of unnecessary component(s) from the suspension L1.

When the replacement solution is used like embodiment 4, in some cases replacement by the replacement solution may be insufficient. In this case, the electric resistance of the suspension L1 is likely to be unstable. However, embodiment 4 includes the sensor electrode 60, 60A, 60B and it is therefore possible to adjust the voltage across the separation electrode 50 in accordance with the measured value of the electric resistance of the suspension L1 by the sensor electrode 60, 60A, 60B, or adjust components of the suspension L1 to be supplied to the flow channel chip 10C1, 10C2, 10C3. If the measured value of the electric resistance of the suspension L1 of the sensor electrode 60, 60A, 60B is an abnormal value, the work for separation can be terminated. Therefore, the flow channel chip 10C1, 10C2, 10C3 enables stabilization of work for separating the specific types of dielectric particles P1 from the suspension L1.

### [2. VARIATIONS]

Embodiments of the present disclosure are not limited to the above embodiment. The above embodiment may be modified in various ways in accordance with designs or the like to an extent that they can achieve the problem of the present disclosure. Hereinafter, some variations or modifications of the above embodiment will be listed. One or more of the variations or modifications described below may apply in combination with one or more of the others.

In one variation, the separation system 1 does not necessarily include the analyzer 13. It is sufficient that the separation system 1 includes the flow channel chip 10 and the measurement device 12.

In one variation, the suspension L1 is not limited to blood. The suspension L1 is not particularly limited if it contains dielectric particles to be separated. The dielectric particles may not be limited to cells described above. Examples of such dielectric particles may include membrane vesicles, microorganism, fungi, spores, viruses, exosomes, and nucleic acids such as DNA or RNA.

In one variation, shapes of the flow channel chips 10, 10A, 10B1, 10B2, 10C1, 10C2, 10C3 may be modified. Especially, the shape of the substrate 10a is not limited to that of the aforementioned embodiments. The shape of the substrate 10a may be appropriately set in accordance with the flow channel 30, 30B, 30C. The flow channel 30, 30B, 30C is not limited to being a straight shape like the aforementioned embodiments but a curved shape. Further, depending on the shape of the flow channel 30, 30B, 30C, the collection part 40 may not be required.

In one variation, the numbers and the shapes, of the separation electrodes 50 may be modified. It is sufficient that the separation electrode 50 can cause the dielectrophoretic force causing the specific type of dielectric particles of the plurality of types of dielectric particles to move in the predetermined direction. Depending on the shape of the separation electrode 50, the separation electrode pads 71, 72 may be omitted.

In one variation, the shape of the sensor electrode 60, 60A, 60B may be modified. It is sufficient that the sensor electrode 60, 60A, 60B can measure the electric resistance of the suspension L1. Depending on the shape of the sensor electrode 60, 60A, 60B, the sensor electrode pads 81, 82 may be omitted.

In one variation, in the flow channel chip 10A, 10C2, the sensor electrode 60A need not be provided to all of the plurality of branch flow channels 32. The sensor electrode 60Amay be provided to at least one of the plurality of branch flow channels 32. In this case, it is possible to measure the electric resistance of the suspension L1 for each branch channel 32. Especially, providing the sensor electrode 60A to not all of the plurality of branch flow channels 32 but one or some of the plurality of branch flow channels 32 enables decreases in a space and cost necessary for placing the sensor electrode(s) 60A.

In one variation, also in the flow channel chip 10, 10A, 10C1, 10C2, 10C3, the sensor electrode 60B may be provided between the inlet 21 and the separation electrode 50 in the flow channel 30, 30B, 30C.

In one variation, the flow channel 30 may include one or more filters between the inlet 21 and the separation electrode 50. The filters may remove particles having sizes smaller than those of the plurality of types of dielectric particles P1, P2 from the suspension L1, for example. The particles having sizes smaller than those of the plurality of types of dielectric particles P1, P2 are particles having sizes smaller than sizes of dielectric particles which are the smallest in size among the plurality of types of dielectric particles P1, P2. The particles having sizes smaller than those of the plurality of types of dielectric particles P1, P2 are small cells such as erythrocytes contained in the suspension L1, for example. The aforementioned filter is capable of separating the particles having sizes smaller than those of the plurality of types of dielectric particles P1, P2 from the plurality of types of dielectric particles P1, P2 in the suspension L1 by a principle of hydrodynamic filtration (HDF).

### [3. ASPECTS]

As apparent from the above embodiment and variations, the present disclosure includes the following aspects. Hereinafter, reference signs in parenthesis are attached for the purpose of clearly showing correspondence with the embodiments only.

The first aspect is a flow channel chip (10; 10A; 10B1; 10B2; 10C1; 10C2; 10C3) including: a substrate (10a) including an inlet (21) for introduction of a suspension (L1) containing a plurality of types of dielectric particles (P1, P2) including a specific type of dielectric particles (P1), and a flow channel (30; 30B; 30C) for allowing a flow of the plurality of types of dielectric particles (P1, P2) of the suspension (L1) introduced from the inlet (21); a separation electrode (50) positioned inside the flow channel (30; 30B; 30C) to cause a dieletrophoretic force for causing the specific type of dielectric particles (P1) of the plurality of types of dielectric particles (P1, P2) to move in a predetermined direction; and a sensor electrode (60; 60A; 60B) positioned inside the flow channel (30; 30B; 30C) to measure an electric resistance of the suspension (L1). This aspect enables stabilization of work for separating the specific types of dielectric particles (P1) from the suspension (L1).

The second aspect is the flow channel chip (10; 10A; 10B1; 10B2; 10C1; 10C2; 10C3) based on the first aspect. In the second aspect, the separation electrode (50) and the sensor electrode (60; 60A; 60B) are separate electrodes. This aspect enables designing the separation electrode (50) and the sensor electrode (60) in accordance with their respective conditions suitable for their respective purposes and thus enables improvement of stabilization of work for separating the specific types of dielectric particles (P1).

The third aspect is the flow channel chip (10; 10A; 10B1; 10B2; 10C1; 10C2; 10C3) based on the first or second aspect. In the third aspect, an area of part positioned inside the flow channel (30; 30B; 30C), of the sensor electrode (60; 60A; 60B) is larger than an area of part positioned inside the flow channel (30; 30B; 30C), of the separation electrode (50). This aspect enables decreasing a variation of a performance in the flow channel chip 10 and additionally enables improvement of a performance of measurement using the sensor electrode (60).

The fourth aspect is the flow channel chip (10; 10A; 10B1; 10B2; 10C1; 10C2; 10C3) based on any one of the first to third aspects. In the fourth aspect, an impedance (or a reactance and a resistance) of the sensor electrode (60; 60A; 60B) is smaller than a lower limit of a measurable range of the electric resistance of the suspension (L1). This aspect enables decreasing a variation of a performance in the flow channel chip 10.

The fifth aspect is the flow channel chip (10; 10A; 10B1; 10B2; 10C1; 10C2; 10C3) based on the fourth aspect. In the fifth aspect, the impedance (or the reactance and the resistance) of the sensor electrode (60; 60A; 60B) is equal to or smaller than one-fifth of the lower limit of the measurable range of the electric resistance of the suspension (L1). This aspect enables decreasing a variation of a performance in the flow channel chip 10 and additionally enables improvement of a performance of measurement using the sensor electrode (60).

The sixth aspect is the flow channel chip (10; 10A; 10B1; 10B2; 10C1; 10C2; 10C3) based on any one of the first to fifth aspects. In the sixth aspect, the sensor electrode (60; 60A; 60B) is a comb electrode including a plurality of teeth (611, 621) arranged along a length direction of the flow channel (30; 30B; 30C) in or on a bottom surface of the flow channel (30; 30B; 30C). This aspect enables stabilization of work for separating the specific types of dielectric particles (P1) from the suspension (L1).

The seventh aspect is the flow channel chip (10; 10A; 10B1; 10B2; 10C1; 10C2; 10C3) based on any one of the first to sixth aspects. In the seventh aspect, the sensor electrode (60; 60A; 60B) is positioned in at least one of a place between the inlet (21) and the separation electrode (50) in the flow channel (30B), or a place on an opposite side of the separation electrode (50) from the inlet (21) in the flow channel (30B). This aspect enables stabilization of work for separating the specific types of dielectric particles (P1) from the suspension (L1).

The eighth aspect is the flow channel chip (10A; 10C2) based on any one of the first to seventh aspects. In the eighth aspect, the flow channel (30; 30C) includes a plurality of branch flow channels (32) connected to a plurality of collection parts (40). The sensor electrode (60A) is provided to at least one of the plurality of branch flow channels (32). This aspect enables measurement of the electric resistance of the suspension (L1) for each branch flow channel (32). In this regard, in the case where the sensor electrode (60A) is provided to all of the plurality of branch flow channels (32), even when there is a distribution of the electric resistance of the suspension (L1) in the width direction of the flow channel (30), it is possible to reduce an effect due to such a distribution of the electric resistance of the suspension (L1) and thus it is possible to conduct more accurate measurement of the electric resistance of the suspension (L1). In contrast, in the case where the sensor electrode (60A) is provided to not all of the plurality of branch flow channels (32) but some of the plurality of branch flow channels (32), it is possible to reduce a space and cost necessary for placing the sensor electrode (60A).

The ninth aspect is the flow channel chip (10; 10C1) based on any one of the first to seventh aspects. In the ninth aspect, the flow channel (30; 30C) includes a plurality of branch flow channels (32) connected to a plurality of collection parts (40). The sensor electrode (60) is provided to span over the plurality of branch flow channels (32). This aspect enables measurement of the electric resistance of the entire suspensions (L1) passing through the flow channel (30) and thus enable more accurate measurement of the electric resistance of the suspension (L1).

The tenth aspect is a separation system (1) including the flow channel chip (10; 10A; 10B1; 10B2; 10C1; 10C2; 10C3) based on any one of the first to ninth aspects, and a measurement device (12) configured to measure the electric resistance of the suspension (L1) based on the output from the sensor electrode (60; 60A; 60B). This aspect enables stabilization of work for separating the specific types of dielectric particles (P1) from the suspension (L1).

The eleventh aspect is the separation system (1) based on the tenth aspect. In the eleventh aspect, the flow channel chip (10; 10A; 10B1; 10B2; 10C1; 10C2; 10C3) is disposable. This aspect enables prevention of contamination and additionally improve usability of the separation system (1).

### INDUSTRIAL APPLICABILITY

The present disclosure applies to flow channel chips and separation systems. Concretely, the present disclosure can apply to a flow channel chip and a separation system for separating a specific type of dielectric particles from a plurality of types of dielectric particles contained in a suspension.

### REFERENCE SIGNS LIST

1 Separation System
10, 10A, 10B1, 10B2, 10C1, 10C2, 10C3 Flow Channel Chip
10a Substrate
12 Measurement Device
21 Inlet
30, 30B, 30C Flow Channel
40-1 First Collection Part (Collection Part)
40-2 Second Collection Part (Collection Part)
50 Separation Electrode
60, 60A, 60B Sensor Electrode
611 First Tooth (Tooth)
621 Second Tooth (Tooth)
L1 Suspension
P1, P2 Dielectric Particle

## Claims

1. A flow channel chip comprising:
a substrate including an inlet for introduction of a suspension containing a plurality of types of dielectric particles including a specific type of dielectric particles, and a flow channel for allowing a flow of the plurality of types of dielectric particles of the suspension introduced from the inlet;
a separation electrode positioned inside the flow channel to cause a dieletrophoretic force for causing the specific type of dielectric particles of the plurality of types of dielectric particles to move in a predetermined direction;
a sensor electrode positioned inside the flow channel to measure an electric resistance of the suspension.

2. The flow channel chip according to claim 1, wherein
the separation electrode and the sensor electrode are separate electrodes.

3. The flow channel chip according to claim 1 or 2, wherein
an area of part positioned inside the flow channel, of the sensor electrode is larger than an area of part positioned inside the flow channel, of the separation electrode.

4. The flow channel chip according to any one of claims 1 to 3, wherein
an impedance of the sensor electrode is smaller than a lower limit of a measurable range of the electric resistance of the suspension.

5. The flow channel chip according to claim 4, wherein
the impedance of the sensor electrode is equal to or smaller than one-fifth of the lower limit of the measurable range of the electric resistance of the suspension.

6. The flow channel chip according to any one of claims 1 to 5, wherein
the sensor electrode is a comb electrode including a plurality of teeth arranged along a length direction of the flow channel in or on a bottom surface of the flow channel.

7. The flow channel chip according to any one of claims 1 to 6, wherein
the sensor electrode is positioned in at least one of a place between the inlet and the separation electrode in the flow channel, or a place on an opposite side of the separation electrode from the inlet in the flow channel.

8. The flow channel chip according to any one of claims 1 to 7, wherein
the flow channel includes a plurality of branch flow channels connected to a plurality of collection parts, and
the sensor electrode is provided to at least one of the plurality of branch flow channels.

9. The flow channel chip according to any one of claims 1 to 7, wherein
the flow channel includes a plurality of branch flow channels connected to a plurality of collection parts, and
the sensor electrode is provided to span over the plurality of branch flow channels.

10. A separation system comprising:
the flow channel chip according to any one of claims 1 to 9; and
a measurement device configured to measure the electric resistance of the suspension based on the output from the sensor electrode.

11. The separation system according to claim 10, wherein
the flow channel chip is disposable.
